# EUROPÄISCHE PATENTSCHRIFT

(11) **EP 1 841 325 B1**
(45) Veröffentlichungstag und Bekanntmachung des Hinweises auf die Patenterteilung: **14.07.2010**
(21) Anmeldenummer: 06829208.5
(22) Anmeldetag: 30.11.2006
(51) Int. Cl.: A23C 9/00, A01K 29/00, A61K 35/20

(54) **VERFAHREN ZUR HERSTELLUNG VON MILCH ODER MILCHERZEUGNISSEN MIT HOHEM MELATONINANTEIL**
METHOD FOR THE PRODUCTION OF MILK OR MILK PRODUCTS HAVING A HIGH MELATONIN CONTENT
PROCEDE DE PRODUCTION DE LAIT OU DE PRODUITS LAITIERS A HAUTE TENEUR EN MELATONINE

(30) Priorität: 13.12.2005 DE 102005059518
(43) Veröffentlichungstag der Anmeldung: 10.10.2007
(73) Patentinhaber: Gnann, Tony, 80802 München (DE)
(72) Erfinder: Gnann, Tony, 80802 München (DE)
(74) Vertreter: Barz, Peter
(86) Internationale Anmeldenummer: PCT/EP2006/011510
(87) Internationale Veröffentlichungsnummer: WO 2007/068361

(56) Entgegenhaltungen:
- WO-A-01/01784
- WO-A-84/00693
- GB-A- 2 387 099
- DATABASE FSTA [Online] INTERNATIONAL FOOD INFORMATION SERVICE (IFIS), FRANkFURT-MAIN, DE; STUTZER D: "Some questions about night milk." XP002425619 Database accession no. 2005-00-p0655 & STUTZER D: "Some questions about night milk." DEUTSCHE MILCHWIRTSCHAFT, Bd. 55, Nr. 22, 2004, Seiten 908-908,
- DATABASE FSTA [Online] INTERNATIONAL FOOD INFORMATION SERVICE (IFIS), FRANkFURT-MAIN, DE; ANONYMOUS: "Swiss regulator turns off the lights on Nachtmilch." XP002425620 Database accession no. 2005-00-p1131 & ANONYMOUS: "SWISS REGULATOR TURNS OFF THE LIGHTS ON NACHTMILCH." NUTRITION BUSINESS, Bd. 10, Nr. 6, 2005, Seiten 3-4,
- DATABASE WPI Week 200513 Derwent Publications Ltd., London, GB; AN 2005-113302 XP002425636 & CN 1 537 438 A (YU N) 20. Oktober 2004 (2004-10-20)
- ERIKSSON L ET AL: "DIURNAL RHYTHM OF MELATONIN IN BOVINE MILK: PHARMACOKINETICS OF EXOGENOUS MELATONIN IN LACTATING COWS AND GOATS" ACTA VETERINARIA SCANDINAVICA, ACTA VETERINARIA SCANDINAVICA, KOPENHAGEN, DK, Bd. 39, Nr. 3, 1998, Seiten 301-310, XP002933605 ISSN: 0044-605X
- GUSTAFSON G M: "EFFECT OF CHANGES IN LIGHT ON HORMONAL SECRETION AND MILK PRODUCTION OF DAIRY COWS IN EARLY LACTATION" ACTA AGRICULTURAE SCANDINAVICA, STOCKHOLM, SE, Bd. 44, 1994, Seiten 160-168, XP002933606 ISSN: 0001-5121
- SARKAR ET AL: "Circadian variations in plasma concentrations of melatonin and prolactin during breeding and non-breeding seasons in yak (Poephagus grunniens L.)" ANIMAL REPRODUCTION SCIENCE, ELSEVIER SCIENCE PUBLISHERS, AMSTERDAM, NL, Bd. 90, Nr. 1-2, November 2005 (2005-11), Seiten 149-162, XP005135477 ISSN: 0378-4320

## Beschreibung

Das Verfahren betrifft ein Verfahren zur Herstellung von Milch mit hohem Melatoningehalt und daraus erhältliche Milcherzeugnisse.

Das Hauptsekretionsprodukt der Epiphyse oder Zirbeldrüse ist das 1958 von Lerner gefundene Indolamin Melatonin, das über Serotonin aus der Aminosäure Tryptophan entsteht. In den Folgejahren wurden die Wirkungen von Melatonin untersucht. Durch orale Verabreichung von Melatonin konnten positive Wirkungen erzielt werden, so dass viele Verwendungsmöglichkeiten in der Humanmedizin und im Bereich der Nahrungsergänzung entwickelt worden sind. Allerdings muss hierzu synthetisches Melatonin aus pharmazeutischer Herkunft verwendet werden, da Melatonin aus bislang natürlichen Quellen nicht in ausreichendem Maße verfügbar war.

Melatonin ist ein hydrophiles Aminosäurenderivat. Es wirkt im Körper als Hormon und Antioxidans. Zahlreiche neurobiologische Funktionen wurden inzwischen beim Menschen festgestellt, wie z.B. als "Anti-Aging-Mittel", Radikalfänger, Regulator der circadianen Uhr und endogenen Induktion von Schlaf, sowie ein Einfluss auf die Reproduktion, das Immunsystem, die Körpertemperatur und die Hirnaktivität. Das Hormon Melatonin wird beim Menschen wie auch bei den Säugetieren von der Zirbeldrüse ausgeschüttet. Bei der Synthese wird die Aminosäure Tryptophan decarboxyliert und hydroxyliert. Aus dem so entstandenen Serotonin wird durch N-Acetylierung und Methylierung Melatonin (= N-Acetyl-5-methoxyltryptamin).

Die Verwendung von mit Melatonin angereicherter Milch oder Milcherzeugnissen daraus gegen den zunehmend absinkenden Melatoninspiegel im Alter wäre z.B. vom wissenschaftlichen Standpunkt aus ein logisches Konzept. Der Melatonintagesspiegel im Blut liegt bei etwa 20 bis 70 pg/ml bei jungen Menschen (20-30 Jahre). Er steigt nachts auf ca. 125 pg/ml an. Dieser Konzentrationsbereich wäre der nach Konsum von Milch oder Milchpulver zu erreichende. Bei oraler Aufnahme unterliegt Melatonin jedoch einem relativ hohen First-pass Mechanismus, d.h. etwa 30% werden von der Leber direkt verstoffwechselt und ausgeschieden und treten daher nicht als wirksamer Spiegel im Blut auf. Es müsste also eine um 30% höhere Menge oral zugeführt werden, um die gewünschte Zielkonzentration zu erreichen.

Melatonin aus natürlichen Quellen steht bis heute nur begrenzt zur Verfügung. Bisher wurden geringste Konzentrationen in wenigen Pflanzenarten gefunden. Es gibt jedoch keine Verfahren zur systematischen Gewinnung und kein natürliches Depot zur Aufbewahrung von melatoninreichen Lebensmitteln. Untersuchungen und Forschungen über die medizinische Wirksamkeit und biologische Verfügbarkeit von natürlich hergestelltem Melatonin wurden bisher nicht veröffentlicht. Inwieweit natürlich gewonnenes sich von dem pharmazeutisch produzierten Melatonin hinsichtlich seiner biologischen Wirkung und Verfügbarkeit unterscheidet, ist bisher nicht hinreichend untersucht worden.

Es ist bekannt, dass Melatonin im Blutplasma von Menschen und Säugetieren in Spuren vorkommt und ständig reproduziert wird. An Blutplasma gebundenes Melatonin eignet sich jedoch nicht für eine Verwendung in der Humanmedizin bzw. als Nahrungs- oder Nahrungsergänzungsmittel.

Hingegen eignet sich für die Gewinnung von natürlichem Melatonin ein mit Hilfe von Blut im Körper von verschiedenen Säugetieren hergestelltes und allgemein bekanntes Lebensmittel, nämlich Milch. Das Melatonin ist dabei insbesondere an das Milcheiweiß gebunden.

Für den Menschen ist bekannt, dass der Wechsel von Lichtbestrahlung für die Steuerung des Tageszeit- und Jahreszeit-bezogenen Verhaltens bedeutsam ist. Die Hell/Dunkel-Zyklen steuern viele Verhaltensweisen des Menschen, einschließlich Winterdepression, Schtaf-/Wach-Rhythmen, Körpertemperatur, Hirnaktivitäten, subjektiver Wachsamkeit und Leistungsfähigkeit. Diese beim Menschen bekannten Einflüsse gelten weitgehend auch für Säugetiere.

Alle Tiere sind dem rhythmischen Wechsel von Tag und Nacht angepasst. So genannte innere Uhren steuern alle wichtigen Lebensfunktionen wie den Stoffwechsel, die Körpertemperatur, das Hormon- und Immunsystem sowie das Verhalten im tagesperiodischen Wechsel. Der 24-Stunden-Rhythmus dieses inneren Schrittmachers wird allerdings nicht von externen Zeitinformationen gesteuert. Dieses sogenannte circadiane System funktioniert auch in Abwesenheit von äußeren Faktoren, entspricht aber nicht exakt einem Tag. Der Ausdruck circadian leitet sich von den lateinischen Ausdrücken "circa" (ungefähr) und "dies" (Tag) ab.

Die Synchronisation des internen Schrittmachers mit dem externen Tag-NachtRhythmus geschieht über Zeitgeber, externe Stimuli, welche Informationen über die Tageszeit an den Körper vermitteln. Der wichtigste Zeitgeber für Säugetiere ist das Licht. Aber auch Faktoren wie Temperatur, Aktivität und soziale Interaktion können den circadianen Rhythmus verschieben. Es gibt viele Hinweise darauf, dass das circadiane System bei Säugetieren durch Licht ausschließlich über die Retina synchronisierbar ist, wobei Informationen über die Lichtverhältnisse im wesentlichen über retinale Photorezeptoren erhalten werden.

Die heute üblichen Haltungsverfahren für laktierende Säugetiere sehen vor, dass sich die Tiere frei bewegen dürfen und sowohl tagsüber wie auch nachts ihre Liege-Fress- und Melkplätze frei aufsuchen können. Die Haltungsräume sind nachts mit meist weißfarbigen Notbeleuchtungen ausgestattet, damit die Tiere Freund und Feind unterscheiden und ihre gewünschten Ziele finden können. Solche herkömmlichen Beleuchtungssysteme reduzieren die Produktion von Melatonin in der Nacht.

In der WO 01/01784 wird ein Verfahren zur Herstellung melatoninreicher Milch beschrieben, bei dem der Tagesrhythmus von Säugetieren in eine helle und eine dunkle Periode unterteilt wird und die Tiere am Ende der dunklen Phase gemolken werden. Die Lichtmenge während der dunklen Periode ist dabei bevorzugt unter 40 lux. Auch in der GB-A-2387099 wird ein Verfahren zur Herstellung melatoninreicher Milch beschrieben, bei dem der Tagesrhythmus von Säugetieren in eine helle und eine dunkle Periode unterteilt wird, wobei die Lichtintensität in der dunklen Periode 50 lux nicht überschreiten soll. Auch wurden Versuche durch Verdunkelung und Verwendung von Schwarzlicht in der dunklen Periode beschrieben.

Den bisher bekannten Verfahren ist gemeinsam, dass in der "dunklen Phase" der Tierhaltung eine möglichst geringe Lichtmenge als zwingend angesehen wird. Dies ist aber mit Schwierigkeiten verbunden, da die Tiere während dieser dunklen Phase aufgrund des fehlenden oder mangelhaften Lichts sich nur schwer oder überhaupt nicht orientieren können, was insbesondere während des Melkvorgangs nicht praktikabel ist. Der Melatoningehalt der Milch wird dadurch negativ beeinflusst.

Insbesondere wenn die Tiere in einem Stall und in großer Anzahl gehalten werden, stellt die fehlende Orientierung ein ernstes Problem dar. Daher sind die vorstehend beschriebenen Verfahren allenfalls für bäuerliche Kleinbetriebe geeignet. Für größere Tierherden in Freilaufsystemen sind diese Verfahren nach dem Stand der Technik kaum praktikabel. Eine marktrelevante industrielle Produktion ist damit kaum möglich.

D. Stutzer, Deutsche Milchwirtschaft 55(22) 908-909, 2004, betrifft Milch mit erhöhten Mengen an Melantonin durch Melken in der Nacht, wobei die physiologische Rolle von Melantonin und das wirtschaftliche Interesse an dieser Milch erörtert werden. In New Nutrition Business 10 (6) 3-4, 2005, wird ebenfalls über Milch mit erhöhtem Melantoningehalt berichtet.

WO 84/00693 beschreibt die Wirkung von Licht mit Wellenlängen von 600 bis 700 nm oder 480 bis 600 nm auf Tiere, wobei u.a. die Verbesserung der Laktation von Säugern durch Rotlicht ausgeführt wird.

CN-A-1537438 betrifft ein Milchpulvemahrungsmittel für die Gesundheitsvorsorge, das aus Milchpulver, Melantonin, Perlweiß und Oligopectose hergestellt wird.

Die Aufgabe der vorliegenden Erfindung bestand daher in der Bereitstellung eines Verfahrens zur Herstellung von Milch mit erhöhtem Melatoningehalt, das den Tieren und auch dem Bedienungspersonal bei Nacht eine ausreichende Orientierung ermöglicht und auch für eine erhöhte Anzahl von Tieren geeignet ist.

Diese Aufgaben konnten überraschenderweise durch ein Verfahren zur Herstellung von Milch mit erhöhtem Melatoningehalt oder Milcherzeugnissen davon gelöst werden, bei dem der Tagesrhythmus von einem oder mehreren weiblichen Säugetieren in eine Tagphase unter einem ersten Lichtregime mit Blaulichtanteil und in eine Nachtphase unter einem zweiten Lichtregime unterteilt wird und das oder die Tiere während der Nachtphase mindestens einmal gemolken werden, um Milch mit erhöhtem Melatoningehalt zu erhalten, das dadurch gekennzeichnet ist, dass während der Nachtphase mindestens eine Lichtquelle für das Lichtregime eingesetzt wird, die Licht im Wellenlängenbereich von 500 nm oder mehr und im wesentlichen kein Licht im Wellenlängenbereich unter 500 nm emittiert. Die Lichtquelle emittiert insbesondere Licht der Farbe gelb, orange, amber oder rot oder einer Mischfarbe davon, wobei rotes Licht besonders bevorzugt ist.

Mit der hier vorgestellten Erfindung wird ein Verfahren beschrieben, bei dem unter Einsatz von zweckmäßigen Lichtregimen der circadiane Rhythmus und die Melatoninsuppression der Tiere so beeinflusst werden, dass eine Milch mit erhöhtem Melatoningehalt erhalten wird. Dabei werden die Tiere in der Nachtphase trotzdem unter einem Lichtregime gehalten, das eine ausreichende Orientierung gewährleistet. Damit kann das Verfahren auch bei einer größeren Anzahl von Tieren eingesetzt werden.

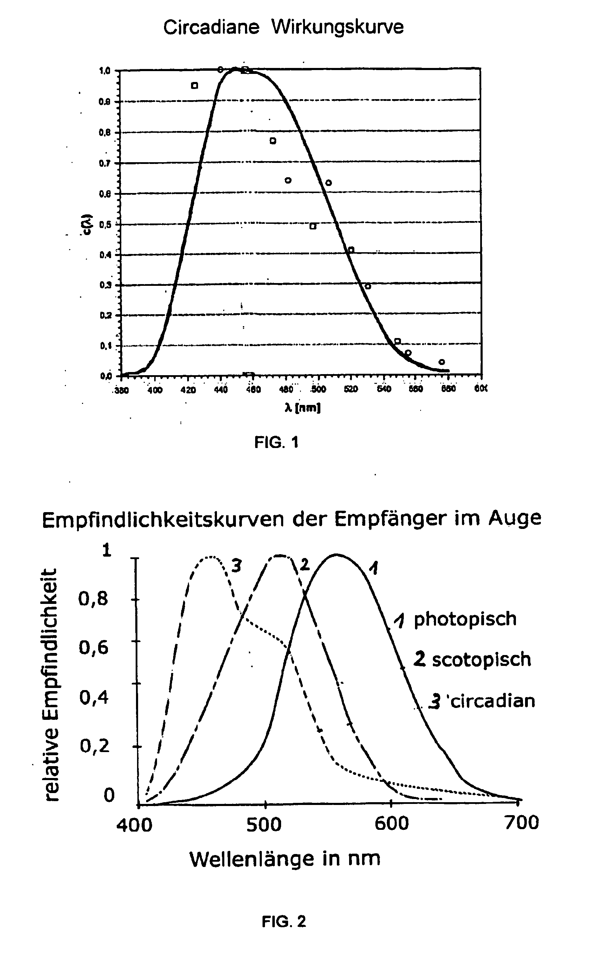

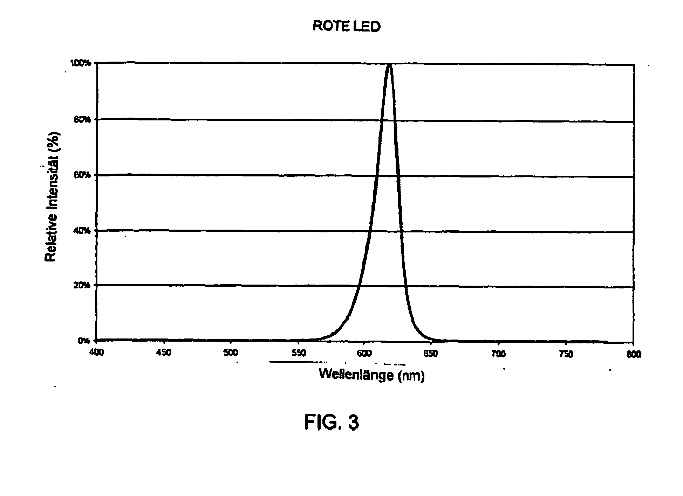

Figur 1 zeigt eine gemittelte circadiane Wirkungskurve in Abhängigkeit von der Wellenlänge des Lichts. Figur 2 vergleicht die Wellenlängenabhängigkeit der photopischen, skotopischen und circadianen Wirkung. Photopisch bezieht sich auf das Sehen bei normaler Helligkeit. Skotopisch bezieht sich auf das Sehen in der Dämmerung oder im Dunkeln. Figur 3 zeigt die Spektralverteilung einer roten LED.

Für die Erzeugung von mit Melatonin hochangereichertem Milcheiweiß eignen sich alle laktierenden Tiere, insbesondere weibliche Tiere ausgewählt aus den Säugetierrassen Schafe, Kühe und Ziegen. Diese drei Tierarten bieten durch ihre physiologischen Voraussetzungen ein ideales wirtschaftliches Verhältnis von Körpergewicht zu Milchertrag. Sie verfügen über ähnliche circardiane Rhythmen und Systeme und natürliche Milchdepots (Euter). Außerdem verfügen sie über weltweit verbreitete Populationen und sind gut verfügbar.

Es werden ein oder mehrere Tiere unter dem erfindungsgemäßen Tagesrhythmus gehalten, bevorzugt mindestens 10, bevorzugter mindestens 50 oder 100 oder sogar mehr als 200 Tiere. Bevorzugt wird eine laktierende Tiergruppe dem Rhythmus nach der Erfindung unterworfen. Die Haltung der ganzen Gruppe unter dem erfindungsgemäßen Tagesrhythmus ist vorteilhaft, da dadurch kein spezieller Eingriff erforderlich ist, die Tiere keine ungewohnte Änderung erfahren und eine bei Absonderung erforderliche unterschiedliche Haltung der Tiere vermieden werden kann.

Wenn nicht anders angegeben, wird hier unter Licht die in das Auge eintreffende optische Strahlung, die eine Sehempfindung hervorruft, verstanden, d.h. Strahlung im Wellenlängenbereich von 380 bis 780 nm. Die Beleuchtungsstärke wird allgemein als Lichtmenge definiert, die auf eine Fläche fällt, und nicht die Quantität des Lichts, die auf die Netzhaut auftrifft. Letzteres ist jedoch für die circadiane Wirkung des Lichts entscheidend. Nicht die Lichtstärke einer Lichtquelle in Lux, sondern die Farbe des Lichts und die Wellenlänge der Lichtfarben sind für die gewünschte Wirkung auf das zur Melatoninproduktion wichtige circadiane System wichtig.

Als Lichtquellen werden hier dementsprechend nur solche angesehen, die im Bereich des sichtbaren Lichts emittieren. Die Spektralverteilung des von Lichtquellen erzeugten Lichts wird in Emissionsspektren deutlich, in denen die Intensität in Abhängigkeit von der Wellenlänge wiedergegeben wird. Häufig wird die relative Intensität angegeben, wobei der höchste Wert im untersuchten Spektrum zu 100% gesetzt wird.

Die Menge des Lichts, seine spektrale Zusammensetzung, räumliche Verteilung sowie Zeitpunkt und Dauer, die für das Sehvermögen von Säugetieren erforderlich sind, unterscheiden sich signifikant von Anforderungen, die sich aus circadianen Funktionsweisen ergeben.

Die Erfindung beruht unter anderem auf der Abhängigkeit der Melatoninsuppression von der spektralen Zusammensetzung der verwendeten Lichtquelle. Durch die Erfindung kann eine optimal angepasste Beleuchtung mit künstlichem Licht für eine maximale Melatoninausbeute erreicht werden. Hierfür wurden die Farbparameter verschiedener Lichtquellen in Bezug auf ihre Wirksamkeit für die Melatoninsuppression untersucht.

Licht ist der Primärerreger für die Steuerung der Melatoninbildung. Der Einsatz von ausgewählten Lichtquellen zur zielgerichteten Wirkung auf circadiane Funktionen ergibt eine durch unterschiedliche Lichtregime gesteuerte Unterdrückung oder Stimulierung der Melatoninsekretion durch die Zirbeldrüse und demzufolge eine erhöhte Melatoninkonzentration im Blutplasma. Der Melatoningehalt im Blutplasma korreliert, etwas unterschiedlich und zeitversetzt, mit der Melatoninkonzentration der Milch.

Es wird eine neue Art und Kombination von Lichtquellen und -farben eingesetzt, da die Quantität der Lichtquelle, das Spektrum der Lichtfarben und deren Wellenlänge, die räumliche Verteilung und der Zeitpunkt und die Dauer der Lichteinwirkung für die circadiane Beeinflussung der Tiere völlig anders sind als die, die für das gewöhnliche Sehen wichtig sind. Es wurde festgestellt, dass es zur Beeinflussung des Melatoninspiegels im Blut von Säugetieren gut und schlecht geeignetes Licht gibt. Die gefundenen Lichtsysteme sind geeignet, sowohl photopische als auch circadiane Effekte von Licht zur Melatoninsuppression präzise zu steuern.

Die Anpassung der physiologisch-psychologischen Prozesse des Körpers an die zeitlichen Umweltbedingungen erfolgt durch die innere Uhr. Bei Ausschaltung von Zeitgebern läuft die innere Uhr frei. So dauert z. B. bei völliger Dunkelheit die freilaufende circadiane Periode des Menschen im Mittel 10 min bis 20 min länger als der 24-Stunden-Tag. Störungen der Synchronisation der inneren Uhr und daraus resultierender fehlender Tagesrhythmik sind für die Melatoninproduktion hochwirksam im negativen Sinne. Die Anpassung der circadianen Tagesperiodik an den aktuellen Tages-Nachtrhythmus erfolgt durch das retinal absorbierte Licht und den Mechanismus der Melatoninsuppression.

Nicht die Beleuchtungsstärke einer Lichtquelle gemessen in Lux, sondern die spektrale Abhängigkeit ist für die Melatoninsuppression entscheidend. Es stellte sich heraus, dass die spektrale Empfindlichkeit der circadianen Photorezeptoren sich im Vergleich zur Hellempfindlichkeitskurve für das Tagessehen vor allem vom kurzwelligen Bereich des sichtbaren Spektrums beeinflussen lassen. Daraus resultiert, dass z.B. Blauanteile des Lichtes bei der Steuerung des circadianen Systems wirksamer sind als andere Spektralfarben.

Folglich ist unter dem Gesichtspunkt der höchstmöglichen Effizienz der Melatoninproduktion die spektrale Empfindlichkeit, d.h. die Abhängigkeit der Effizienz von der Wellenlänge, von großer Bedeutung. Es ist dabei zwischen der photopischen bzw. scotopischen Wirkung und der circadianen Wirkung, also der Wirkung auf die Melatoninproduktion, des Lichts zu unterscheiden.

In Figur 1 ist eine circadiane Wirkungskurve in Abhängigkeit von der Wellenlänge des Lichts gezeigt. Figur 2 zeigt Empfindlichkeitskurven für die photopische, scotopische und circadiane Wirkung in Abhängigkeit von der Wellenlänge.

Photopische und circadiane Lichtausbeuten unterscheiden sich je nach Art der gewählten Lichtquelle gravierend. Die Bemessung der Lichtausbeute in Lux oder Lumen pro Watt ist deshalb nicht geeignet zur Beurteilung der Melatoninsuppression. Die maximale Wirksamkeit der Melatoninsuppression erfolgt bei Licht in Wellenlängen von ca. 450 bis 470 nm. Diese Wellenlängen sind im Spektralfarbenbereich des Sonnenlichts enthalten und in der künstlichen Lichtfarbe "blau" vorhanden. Die photopische und circadiane Wirkung verschiedener Lichtquellen ist in nachfolgender Tabelle beispielhaft wiedergegeben.

| Lichtquelle | Lichtausbeute (photopisch) (Im/W) | Lichtausbeute (circadian) (Im/W) | Verhältnis der Lichtausbeuten circadian/photopisch |
|---|---|---|---|
| 3000 K Leuchtstoff | 87 (1,00) | 149 (1,00) | 1,00 |
| seltene Erden | | | |
| 4100 K Leuchtstoff | 87 (1,00) | 275 (1,85) | 1,85 |
| seltene Erden | | | |
| 7500 K Leuchtstoff | 65 (0,75) | 285 (1,91) | 2,56 |
| seltene Erden | | | |
| Schwefel-Scandium | 108 (1,24) | 300 (2,02) | 1,63 |
| Metalldampf | | | |
| Schwefelhochdruck | 127 (1,46) | 115 (0,07) | 0,53 |
| LED rot (630 nm) | 44 (0,51) | 2 (0,02) | 0,03 |
| LED gelb (590 nm) | 36 (0,41 | 10 (0,07) | 0,17 |
| LED grün (520 nm | 25 (0,29) | 88 (0,59) | 2,06 |
| LED blau (460 nm) | 11 (0,13) | 681 (4,59) | 36,2 |
| LED weiß (460 nm + Leuchtstoff) | 18 (0,21) | 90 (0,60) | 2,91 |
| Tageslicht (6500 K) | - | - | 2,78 |

Die photopische Lichtausbeute, bezogen auf eine Leuchtstofflampe mit 3000 K (Kelvin), beträgt bei einer LED in der Spektralfarbe rot 44, bei der LED in der Farbe blau hingegen 11. Die circadiane Lichtausbeute, bezogen auf eine Leuchtstofflampe mit 3000 K, beträgt bei der LED in der Farbe rot 2, bei einer LED in der Farbe blau hingegen 681.

Für den Melatoningehalt ist nur relevant, dass farbige Lichtquellen mit maximaler Lichtausbeute im kurzwelligen Bereich die circadiane Wirkung maximieren und farbige Lichtquellen im langwelligen Bereich diese minimieren. Beispielsweise haben eine blaue LED (Maximum ca. 460 nm) und eine rote LED (Maximum ca. 630 nm) etwa die gleiche photopische Beleuchtungsstärke. Die circardiane Lichtausbeute dieser beiden Farben unterscheidet sich aber ungefähr im Verhältnis 1200:1.

Das Verhältnis der relativen circadianen zu photopischen Lichtausbeuten bezogen auf eine Leuchtstofflampe mit 3000 K, erreicht bei einer LED in der Farbe rot die höchstmögliche Effizienz zur Förderung der Melatoninbildung. Einen abgeschwächte, aber hinreichende Wirkung erreicht man mit einer LED in den Farben amber, orange oder gelb bzw. Mischfarben dieser Spektren oder durch den Einsatz einer Natriumdampflampe mit der Farbe gelb.

Die Tagphase ist eine durch gezielten Lichteinfluss gesteuerte Phase der maximalen Melatoninsuppression, vorzugsweise tagsüber unter Ausnutzung der natürlichen Tageshelligkeit, während die Nachtphase eine durch gezielten Lichteinfluss gesteuerte Phase der maximalen Unterdrückung der Melatoninsuppression ist, vorzugsweise nachts unter Ausnutzung der natürlichen Dunkelheit.

Demgemäß wird der Tagesrhythmus der Tiere in eine Tagphase unter einem Lichtregime und eine Nachtphase unter einem anderen Lichtregime unterteilt. Insbesondere handelt es sich bei dem Lichtregime der Tagphase um ein Lichtregime mit Blaulichtanteil. In der Nachtphase weist das eingesetzte Licht im wesentlichen keinen Blaulichtanteil auf.

Die erfindungsgemäß verwendete Lichtregime sind hinsichtlich Intensität und zeitlichen Einsatz prinzipiell beliebig steuerbar. Die jeweiligen Phasen können beliebig verkürzt, verlängert, nach vorne oder hinten verschoben werden. Allerdings lassen sich circadiane Rhythmen nur sehr langsam umstellen, da das circadiane System sehr träge reagiert. Daher kann es zweckmäßig sein, den Tagesrhythmus langsam, z.B. in mehreren Stufen, auf den gewünschten Tagesrhythmus einzustellen und/oder mit der eigentlichen Milchgewinnung mit erhöhtem Melatoningehalt erst nach einer Gewöhnungsphase, z.B. einigen Tagen, zu beginnen.

Ungeachtet zeitlich beliebig steuerbarer Lichteinsätze ist es vorteilhaft, dass die Tagphase z.B. etwa 8 bis 22 Stunden, zweckmäßigerweise etwa 12 bis 21 Stunden und bevorzugt etwa 14 bis 20 Stunden dauert. Eine günstige Dauer beträgt z.B. etwa 17 Stunden plus/minus 1 Stunde oder mehr. Die Nachtphase kann z.B. etwa 2 bis 16 Stunden, zweckmäßigerweise etwa 3 bis 12 Stunden und bevorzugt etwa 4 bis 10 Stunden dauern. Eine besonders günstige Dauer beträgt z.B. etwa 6 Stunden plus/minus 1 Stunde oder weniger.

Die Tagphase ist dabei die Phase der Suppression der Melatoninbildung, während in der Nachtphase die Suppression unterdrückt wird. Um die tageszeitlichen Hell- und Dunkelphasen zu nutzen, sollten die Tag- und Nachtphasen daran orientiert werden. Dies ist aber nicht prinzipiell notwendig, sondern nur aufgrund praktischer Erwägungen zweckmäßig. Die Tagphase (Suppressionsphase) könnte z.B. in die Zeit von ca. 5.00 Uhr bis 22.00 Uhr und die Nachtphase (Unterdrückung der Suppressionsphase) in Zeit ab ca. 22.00 Uhr bis ca. 5.00 Uhr gelegt werden. Selbstverständlich können die Phasen aber auch in andere Zeitintervalle gelegt werden.

Der Übergang von einer Phase in die andere Phase sollte jeweils bevorzugt einen Helligkeits- und Dunkelheitsübergang simulieren, welcher die natürlichen Lichtübergänge zu Nacht und Tag und umgekehrt nachempfindet. Jede Störung der gewohnten Rhythmen beeinträchtigt die Melatoninsekretion. Ein solcher Übergang kann z.B. ca. 30 min bis 1,5 Stunden, bevorzugt ca. 1 h, dauern. Die Übergangszeit kann dann jeweils zur Hälfte der Tag- und Nachtphase zugerechnet werden.

Die Tagphase und die Nachtphase sind jeweils durch ein unterschiedliches Lichtregime gekennzeichnet. Beide Lichtregime können durch künstliches Licht gesteuert werden; in der Tagphase bietet sich auch der Einsatz von normalem Sonnenlicht an. In der Tagphase sind die Tiere insbesondere einem Lichtregime mit Blaulichtanteil unterstellt, der eine hohe photopische und circadiane Wirkung aufweist. Blaulicht ist Licht, das im Wellenlängenbereich von ca. 440 bis 490 nm liegt. Eine maximale Melatoninsuppression kann durch Auslauf der Tiere ins Sonnenlicht oder durch den Einsatz von Lichtquellen mit hoher circadianer Wirkung erreicht werden. Erfindungsgemäß bevorzugt werden Vollspektrumlampen (ca. 375 - 725 nm), welche dem Sonnenlicht (ca. 290 - 770 nm) am ähnlichsten sind und neben dem bekannten Farbenspektrum des Regenbogens wichtiges UV-Licht beinhalten. Eine mehrstündige, das Sonnenlicht simulierende Bestrahlung bewirkt ebenfalls eine maximale Melatoninsuppression durch eine starke Wirkung auf das circadiane System.

Vollspektrumlampen sind im Handel erhältlich. Beispiele sind Lichtfarbe 940 Weiß von Osram oder Biolux von Osram mit der Lichtfarbe 965. Letztere ist bevorzugt, da sie das Sonnenspektrum annähernd simuliert. Ähnliche Vollspektrumlampen werden auch von anderen Herstellern angeboten.

Neben Sonnenlicht und Vollspektrumlampen können auch andere Lichtquellen eingesetzt werden, die durch hohe circadiane Wirkung die Produktion von Melatonin unterdrücken. Möglich wäre z.B. der Einsatz von blauem Licht (Wellenlänge ca. 460 nm) oder anderen Lichtquellen mit Blaulichtanteil, mit denen eine hohe Melatoninsuppression erreicht wird. Beim Einsatz von Blaulicht ist jedoch die wellenlängenabhängige Gefahr der thermischen Netzhautgefährdung zu beachten.

Bei einem Lichtregime durch künstliches Licht wie Vollspektrumlampen kann die Tagesphase auch beliebig verlängert oder verkürzt werden. Die Milchleistung der laktierenden Tiere wird durch Verlängerung der Helligkeitsphase signifikant erhöht.

In der Nachtphase werden die Tiere unter ein Lichtregime gestellt, das die Melatoninsuppression unterdrückt und damit die Melatoninbildung fördert. Die maximale Unterdrückung der Melatoninsuppression würde prinzipiell durch natürliche Dunkelheit (Fehlen von Licht) erreicht. Allerdings ergibt sich dadurch der Nachteil, dass eine Orientierung nicht mehr möglich ist. Vor allem beim Melkvorgang ist dies nicht praktikabel. Zudem bewirkt die Orientierungslosigkeit Stress bei den Tieren, vor allem wenn sie in großer Zahl und in begrenzter Umgebung gehalten werden. Auch dies beeinträchtigt die Melatoninbildung.

Schwarzlichtlampen sind UV-Lampen (ca. 345-400 nm), welche das circadiane System nur wenig beeinflussen, aber wegen ihrer geringen photopischen Lichtausbeute für die Nachtphase nicht geeignet sind, insbesondere in Freilaufsystemen, da mit ihnen wegen der geringen Belichtungsstärke keine ausreichende Orientierung erreicht werden kann. Schwarzlichtlampen können neben UV-Licht auch sichtbares Licht im Blaubereich emittieren.

Es wurde überraschenderweise festgestellt, dass diese Nachteile überwunden werden können, wenn zur Überwindung der Dunkelheit Lichtquellen verwendet werden, die Licht im Wellenlängenbereich von 500 nm oder mehr emittieren und im Wellenlängenbereich unter 500 nm im wesentlichen kein Licht emittieren, so dass die Lichtquelle insbesondere Licht der Farbe gelb, orange, amber oder rot oder einer Mischfarbe davon emittiert. Die Lichtquelle weist daher im Wellenlängenbereich des sichtbaren Lichts ein Emissionsspektrum auf, das den höchsten Wert mit einer relativen Intensität von 100% bei einer Wellenlänge von 500 nm oder mehr zeigt.

Dass die Lichtquelle im wesentlichen kein Licht mit einer Wellenlänge unter 500 nm emittiert bedeutet dabei insbesondere, dass im Emissionsspektrum des sichtbaren Lichts unter 500 nm jeder messbare Wert, falls überhaupt vorhanden, eine relative Intensität von weniger als 15%, bevorzugt weniger als 10% und besonders bevorzugt unter 5 bzw. unter 3% aufweist. Bevorzugt emittiert die eingesetzte Lichtquelle im wesentlichen kein Licht im Wellenlängenbereich unter 520 nm und bevorzugter unter 540 nm. Besonders bevorzugt emittiert die eingesetzte Lichtquelle kein Licht im Wellenlängenbereich unter 500 nm und insbesondere unter 520 nm und noch bevorzugter unter 530 nm.

Als Lichtquellen können z.B. übliche Lampen, wie z.B. Temperaturstrahler, Kontiniumstrahler, Linienstrahler, Gasentladungslampen, verwendet werden, die einen Monochromator enthalten, so dass im wesentlichen kein Licht mit einer Wellenlänge unter 500 nm emittiert wird. Beispiele für Monochromatoren sind Prismas, Beugungsgitter und optische Filter. Als Filter eignen sich z.B. Interferenzfilter, Bandpassfilter oder Langpassfilter, die kurzwellige Bereiche sperren. Solche Filter sind z.B. von Schott erhältlich. Auf diese Weise können Rotlichtlampen hergestellt werden. Rotlichtlampen mit einer ausreichenden Sperrung von Blaulichtanteilen können in der vorliegenden Erfindung eingesetzt werden.

Solche Lichtquellen, die mit Filtern oder anderen Monochromatoren arbeiten, besitzen aber auch einige Nachteile. Zum einen werden Wellenlängenbereiche nicht völlig abgeschnitten, sondern zunehmend reduziert. Ferner führen Fehler in der Sperrung zu kleinen Peaks bei anderen Wellenlängen oder Oberwellen im Durchlassbereich, so dass auch im Bereich unter 500 nm geringe Lichtanteile vorliegen können. So sind Rotlichtlampen nicht reinfarbig rot, sondern können zusätzlich geringe Anteile weitere Spektralfarben beinhalten. Außerdem wird ein Teil des erzeugten Lichts nicht zur Belichtung verwendet, sondern herausgefiltert. Dies erhöht den Energieverbrauch.

Es werden daher bevorzugt Lichtquellen eingesetzt, die keinen Monochromator erfordern. Dementsprechend werden bevorzugt keine Temperaturstrahler eingesetzt. Vorzugsweise werden Lumineszenzstrahler als Lichtquelle verwendet. Lumineszenzstrahler können sogenannte Linienstrahler oder monochromatische Strahler sein. Beispiele für Lumineszenzstrahler sind Gasentladungslampen und Leuchtdioden. Als Lichtquelle wird daher bevorzugt ein Lumineszenzstrahler verwendet, der im wesentlichen kein Licht mit einer Wellenlänge unter 500 nm oder gar kein Licht mit einer Wellenlänge unter 500 nm emittiert.

Das Emissionsspektrum der Lichtquelle im Wellenlängenbereich des sichtbaren Lichts hat vorzugsweise mindestens ein Maximum über 550 nm, bevorzugter mindestens ein Maximum über 570 nm und noch bevorzugter über 600 nm. Die Lichtquelle hat bevorzugt kein Maximum unter 550 nm, bevorzugter unter 570 nm und noch bevorzugter unter 600 nm im sichtbaren Wellenlängenbereich mit einer relativen Intensität von mehr als 5%.

Es hat sich gezeigt, dass es in der Nachtphase wichtig ist, Licht zu verwenden, bei dem Licht mit Wellenlängen von weniger als 500 nm, besser von weniger 520 nm und noch besser von weniger als 550 nm minimiert und bevorzugt im wesentlichen vollständig oder ganz vermieden wird. Dies ist mit Lichtquellen, die ein kontinuierliches Spektrum zeigen, möglich, wenn eine geeignete Filterung erfolgt. Lumineszensstrahler, wie LED und NDL, sind aber bevorzugter, da sie im Gegensatz zu Temperaturstrahlern ein schmalbandiges Spektrum ausstrahlen und keine Filterung erfordern. Durch das erfindungsgemäße Verfahren kann eine bessere Orientierung der Tiere wegen der weit höheren photopischen Wirkung der eingesetzten Lichtquellen, insbesondere der Lumineszensstrahler, erreicht werden.

Eine geeignete Lichtquelle ist z.B. eine Natriumdampflampe (NDL). NDL sind Gasentladungslampen, welche sich durch hohe photopische Lichtausbeuten auszeichnen und monochromatisches gelbes Licht mit einer Wellenlänge von etwa 589 bis 590 nm emittieren. Die Beleuchtung mit NDL eignet sich zur sicheren Erkennung von Objekten und Hindernissen. Das gelbe Licht soll sich auch weniger anziehend auf Insekten auswirken.

Besonders geeignete Lumineszensstrahler sind Leuchtdioden, die auch als LED (light emitting diode) bezeichnet werden. LEDs sind sehr effiziente Lichtquellen. Sie zeigen gewöhnlich ein relativ schmalbandiges Signal mit einem Maximum im Emissionsspektrum, wie z.B. in Figur 3 gezeigt. Mit LED-Lampen kann der gewünschte Wellenlängenbereich gezielt eingestellt werden und sie besitzen gleichzeitig eine genügend große photopische Wirkung, so dass die Tiere sich bei Beleuchtung mit diesen Lichtquellen gut orientieren können.

Als Lichtquelle für die Nachtphase werden Lichtquellen ausgewählt, deren circadiane Lichtausbeute gering ist. Primär ist die Wahl der richtigen Lichtfarben. Blaue LEDs oder Weißlicht-LEDs sind wegen des Blaulichtanteils ungeeignet. Ideale Lichtfarben sind rot, weniger gut, jedoch auch noch möglich sind amber (auch bernsteinfarben oder "superorange") (z.B. Maximum ca. 612 nm), orange (z.B. Maximum ca. 605 nm) oder gelb (z.B. Maximum ca. 585 nm) sowie Mischfarben dieser Spektren. Gelbes Licht kann auch durch eine NDL erzeugt werden. Bevorzugt sind rote Leuchtdioden (z.B. Maximum ca. 630 nm; einschließlich "Ultrarot" bei einem Maximum von ca. 660 nm), deren photopische Lichtausbeute trotz minimaler circadianer Wirkung sehr hoch ist und die sich deshalb für den Nachtbetrieb ideal eignen. Außerdem sind LEDs die einzigen Leuchtkörper, die rotes Licht in Reinfarbe liefern. Solche LEDs sind handelsüblich und überall erhältlich.

Beispiele für im Handel erhältliche LEDs, die für die Erfindung zweckmäßig sind, sind z.B. Lumileds® Luxeon red 1 Watt, Lumileds® Luxeon Star/O red 1 Watt oder SOUL R32 red 1 Watt.

Es ist nicht notwendig, dass die Lichtquelle während der ganzen Nachtphase eingesetzt wird. Sie werden aber insbesondere zumindest während des Melkvorgangs eingesetzt, da dann die Notwendigkeit der Orientierung bei den Tieren und Bedienungspersonal am größten ist. Bevorzugt ist die Lichtquelle während mindestens ein Drittel oder mindestens die Hälfte der Dauer der Nachtphase im Einsatz. Da die eingesetzte Lichtquelle, vor allem die Lumineszenzlampe, praktisch keine negative Auswirkung auf die Melatoninbildung hat und damit gleichzeitig eine bessere Orientierung für Tiere und Bedienungspersonal möglich ist, werden die Lichtquellen besonders bevorzugt im wesentlichen während der ganzen oder in der ganzen Nachtphase eingesetzt. Die Lichtquelle, insbesondere die LED-Lampe, ist während der Nachtphase in der Regel mindestens 1 Stunde, bevorzugt mindestens 2 Stunden, bevorzugter mindestens 5 Stunden und noch bevorzugter mindestens 6 Stunden im Einsatz.

Ansonsten sollten in der Nachtphase keine anderen Lichtquellen eingesetzt werden. Allerdings ist auch keine Totalabdunklung notwendig. Die natürliche Dunkelheit der Nacht ist in Kombination mit der oder den Lichtquellen für die Nachtphase durchaus zweckmäßig. Insbesondere ist im wesentlichen keine Beleuchtung mit Licht mit Blaulichtanteil (insbesondere von 450 bis 470 nm) vorhanden.

Es ist zu bedenken, dass es sich um natürliche Systeme handelt, so dass kurzfristige Störungen des Systems (Unwetter, Lichtausfall usw.) und Variationen (Anpassung an die Jahreszeit durch Verschiebung der Tag- und Nachtphase) möglich sind und vom Verfahren der Erfindung umfasst werden.

Damit die Lichtregime der Tag- und Nachtphase erfüllt werden, sind die Lichtquellen sowohl in Höhe wie Häufigkeit bevorzugt so zu installieren, dass sich die Lichtwirkung in allen den Tieren zugänglichen Bereichen entfalten kann. Bei Einsatz von Sonnenlicht sind die den Tieren zugänglichen Bereiche natürlich entsprechend zu wählen.

Eine Bestimmung der Lichtausbeute in Lux zwecks Unterdrückung bzw. Stimulierung der Melatoninsuppression ist im Hinblick auf die Melatoninproduktion praktisch irrelevant, da Eigenschaften des eingesetzten Lichts sowohl nach den relevanten Aspekten für das circadiane System als auch für das visuelle System zu berücksichtigen sind. Nicht die Stärke einer Lichtquelle in Lux, sondern die Farbe des Lichts und die Wellenlänge der Lichtfarben sind für die gewünschte Wirkung auf das zur Melatoninproduktion wichtige circadiane System bestimmend.

Überraschenderweise kann die Umgebung der Tiere mit den erfindungsgemäß eingesetzten Lichtquellen in der Nachtphase sogar relativ hell beleuchtet werden, ohne dass dies zu einer signifikanten Reduzierung der Melatoninkonzentration in der Milch führt. Dies steht im Gegensatz zum bisherigen Stand der Technik, in dem man von der Notwendigkeit einer möglichst dunklen Umgebung ausging. Dies hat den entscheidenden Vorteil, dass die Orientierung wesentlich leichter ist und die Beleuchtung ohne weiteres während der ganzen Nachtphase eingeschaltet sein kann.

Die Beleuchtungsstärke, die durch die in der Nachtphase verwendeten Lichtquellen erhalten wird, kann bevorzugt mehr als 50 lux, bevorzugter mehr als 100 lux und besonders bevorzugt mehr als 250 lux betragen. Die Beleuchtungsstärke kann z.B. 500 lux und mehr betragen. Die Beleuchtungsstärke kann mit üblichen Luxmetern gemessen werden. Für Linienstrahler wie LED können für genauere Messungen Spektroradiometer verwendet werden. Die Beleuchtungsstärke bezieht sich auf die in das Auge des Tieres eintreffende Strahlung. Somit wird die angegebene Beleuchtungsstärke auf Augenhöhe der Tiere gemessen. So wird die angegebene Beleuchtungsstärke bei Kühen bei einem Bodenabstand von etwa 1,50 m und bei Ziegen bei einem Bodenabstand von etwa 50 cm gemessen.

Bei der Beleuchtungsstärke ist aber zu berücksichtigen, dass langwelliges Licht wie hier definiert auch eine circadiane Wirkung aufweist, wenn auch eine äußerst geringe, die in Richtung kürzerer Wellenlängen zunimmt. Bei hohen Beleuchtungsstärken kann diese circadiane Wirkung Einfluss auf den Melatoningehalt haben. Es ist daher insbesondere bei Beleuchtungsstärken von mehr als 50 lux bevorzugt, eine Lichtquelle zu verwenden, die Licht mit einem Wellenlängenmaximum über 620 nm emittiert, wie eine rote LED.

Die betreffenden Tiere werden mindestens einmal in der Nachtphase gemolken. Je nach Zahl der zu melkenden Tiere wird der Beginn des Melkvorgangs so gelegt, dass er bis zum Ende der Nachtphase abgeschlossen ist. Der Melkvorgang beginnt z.B. zweckmäßigerweise etwa in der Mitte der Nachtphase, insbesondere wenn eine größere Anzahl von Tieren zu melken ist, um alle Tiere in der Nachtphase melken zu können. Die dabei gewonnene Milch besitzt einen erhöhten Melatoningehalt. Das Hormon Melatonin wird in der Leber hauptsächlich zu 6-Sulphatoxymelatonin umgewandelt und über die Nieren ausgeschieden. Die Halbwertszeit beträgt weniger als etwa 60 Minuten. Deshalb muss zur Erhaltung des Melatoninpegels in der Milch die Unterdrückung der Melatoninsuppression bis zum Ende des Melkvorgangs des Tieres aufrechterhalten werden, d.h. der Melkvorgang erfolgt in der Nachtphase mit Beleuchtung durch die Lichtquelle.

Je stärker und länger die Suppression ist, desto höher ist danach der in der Milch enthaltene Melatoninpeak. Daher kann durch verkürzte Nachtphasen unter einem Lichtregime mit möglichst geringer circadianer Wirkung und Melken am Ende der Nachtphase eine Milch mit höherer Melatoninkonzentration gewonnen werden. Beispielsweise eignen sich verlängerte Tagphasen von 16 h und vorzugsweise mehr als 18 h, um in der Nachtphase einen erhöhten Melatoninpeak zu erhalten. Die Lichtexposition für die Tagphase kann dann unmittelbar nach dem Melkvorgang erfolgen.

Die Tiere können natürlich mehr als einmal am Tag beliebig oft gemolken werden, z.B. zweimal oder häufiger. Bevorzugt wird auch wenigstens einmal während der Tagphase gemolken. Da diese Milch keinen erhöhten Melatoningehalt aufweist, wird sie auch getrennt von der in der Nachtphase gesammelten Milch mit erhöhtem Melatoningehalt verwertet.

Die während der Nachtphase gesammelte Milch wird vorzugsweise rasch auf unter 10°C, z.B. 3 bis 8°C, gekühlt. Rasch bedeutet hier z.B. innerhalb von 2 Stunden oder weniger. Die Milch kann zu allen bekannten Milcherzeugnissen auf übliche Weise weiterverarbeitet werden, wobei Milcherzeugnisse mit einem erhöhten Melatoningehalt erhalten werden. Die aus Milch erhältlichen Milcherzeugnisse wie Milchpulver und die Verfahren zur Herstellung sind gut bekannt. Eine allgemeine Beschreibung findet sich z.B. in Ullmanns, Encyklopädie der technischen Chemie, 4. Aufl., Bd. 16, S. 689 ff. Beispiele für Milcherzeugnisse sind Milchpulver, Käse, Joghurt, Quark und Molkeprodukte. Bevorzugt wird die Milch durch Trocknung in Milchpulver mit hohem Melatoningehalt überführt. Hierfür können die allgemein bekannten Verfahren verwendet werden. Das Hormon Melatonin bindet sich an die Eiweißmoleküle der Milch und wird durch Druck, Wärme oder Gefrierbehandlung nicht zerstört.

Die Milch oder die Milcherzeugnisse, insbesondere das Milchpulver, können für die üblichen Anwendungen eingesetzt werden, insbesondere als oder für Nahrungsmittel, Nahrungsergänzungsmittel und Arzneimittel. Durch weitere Bearbeitung der Milch in Form von Fettreduzierung oder Lactoseentzug kann der relative Anteil von Melatonin am Endprodukt (z.B. Milch, Voll- oder Magermilchpulver) weiter gesteigert werden.

Normale Tagesmilch enthält Melatoninwerte von ca. 1,5 - 3 pg/ml. Die nach dem erfindungsgemäßen Verfahren hergestellte Milch enthält in der Regel mindestens die 2-fache Menge oder sogar bis zum 10-fachen oder mehr. Sie kann z.B. einen Melatoningehalt von mehr als 10 pg/ml, z.B. 15 - 50 pg/ml, aufweisen. Die mit Melatonin angereicherte Milch, die nach dem Verfahren der Erfindung erhalten wird, kann so verarbeitet werden, dass die Konzentration des an die Moleküle des Milcheiweißes gebundenen Hormons Melatonin im Endprodukt eine signifikante Steigerung um das bis zu 350-fache der normal vorhandenen Melatoninkonzentration der Milch erfährt.

Das erhältliche Milchpulver hat z.B. eine Melatoninkonzentration von über 100 pg/g, bevorzugt über 150 pg/g und bevorzugter über 200 pg/g. Die Melatoninkonzentration kann z.B. gegebenenfalls bis zu 1.000 pg/g betragen. Ein markfähiges Endprodukt in Form von Milchpulver, das gemäß der Erfindung erhalten wird, enthält z.B. eine Melatoninkonzentration von etwa 200 bis 500 pg/g. Mit einer derartig gesteigerten Konzentration sind vielfältige Verwendungsmöglichkeiten im medizinischen Bereich und als Nahrungsmittel oder Nahrungsergänzungsmittel möglich. Das Milchpulver kann z.B. mit oder ohne verträgliche Träger z.B. als Pulver, Kapsel, Lösung oder Tablette bereitgestellt werden. Es kann mit weiteren geeigneten Additiven und/oder Wirkstoffen, wie Nährstoffen, z.B. Vitaminen oder Mineralien, oder pharmazeutischen Wirkstoffen gemischt werden.

Das Verfahren nach der Erfindung ist für eine nachhaltige und industrielle Herstellung von natürlichem Melatonin geeignet, besonders auch für die Produktion in Betrieben mit über 200 Tieren. Das natürliche Melatonin ist dabei an Milcheiweiß gebunden. Damit kann natürliches Melatonin, gebunden an Milcheiweiß, in hohen Mengen und auf einfache Weise hergestellt werden, bevorzugt in Form von Milchpulver.

Durch das Verfahren der Erfindung lassen sich u.a. auch folgende Ziele erreichen:
1. Die natürlichen circardianen Rhythmen der Tiere werden unterstützt und dem Körper tageszeitliche Informationen signalisiert.
2. Durch Installation und Steuerung der erfindungsgemäß verwendeten Lichtquellen in den Aufenthaltsräumen der Tiere wird die Produktion des Hormons Melatonin im Blutserum und damit auch in der Milch derart angeregt, dass die Konzentration von Melatonin in der Milch auf das Vielfache der üblichen Konzentration ansteigt. Im Endprodukt werden im Herdendurchschnitt Melatoninwerte von über 200 pg/g Milchpulver erreicht, Einzeltiere erreichen Werte von über 500 pg/g.
3. Durch die zeitlich gezielte Verwendung der vorgenannten Lichtquellen werden die täglichen Hell- und Dunkelphasen gezielt so manipuliert, dass die Melatoninsekretion der Zirbeldrüse maximal unterdrückt bzw. maximal stimuliert wird, so dass in einem genau bestimmbaren Zeitraum des Tages eine höchstmögliche Melatoninkonzentration in der Milch erreicht und durch zeitgenaues Melken der Tiere Milch mit einer vielfach erhöhten Melatoninkonzentration gewonnen wird.
4. In den Aufenthaltsräumen der Tiere können die bisher üblichen Notbeleuchtungen durch weißes Licht (z.B. Glüh-, Neon- oder Leuchtstofflampen) vermieden werden.
5. Eine Verdunkelung der Haltungsräume der Tiere zur Vermeidung von Lichteinfall ist nicht erforderlich, da die natürliche Dunkelheit der Nacht und der Einsatz der vorgenannten Lichtquellen ausreicht, die Melatoninsuppression zu unterdrücken.

### Beispiele

Es wurde die Beeinflussung von Milchkühen durch verschiedene Lichtquellen im Hinblick auf den erzielbaren Melatoningehalt untersucht. Die Untersuchung erfolgte an einer größeren Herde, wobei das Lichtregime der Tages- bzw Nachtphase in den Beispielen 1 bis 3 variiert wurde. Während der Nachtphase wurden zufällig ausgesuchte Milchkühe gemolken und der Melatoningehalt in der Milch bestimmt.

Das gewählte Lichtregime und die ermittelten durchschnittlichen Melatoningehalte in der Milch sind in folgender Tabelle zusammengefasst.

| | Beispiel 1 | Beispiel 2 | Beispiel 3 (Vergleich) |
|---|---|---|---|
| Lichtregime Tagphase | künstliche Tagesbeleuchtung mit weißem Licht, 16 h | natürliches Licht | natürliches Licht |
| Lichtregime Nachtphase | nur Licht von roten Leuchtdioden** | nur Licht von roten Leuchtdioden** | weißes Notlicht |
| Melatonin* | 20,35 | 10,25 | 4,57 |

| | | | |
|---|---|---|---|
| * mittlerer Melatoningehalt der Milch (pg/ml), ** Wellenlänge 600 bis 640 nm | | | |

Es ist unmittelbar ersichtlich, dass durch das erfindungsgemäße Verfahren ein deutlich erhöhter Melatoningehalt erreicht werden kann. Einzelheiten zum Melkschema in den Beispielen sind in den folgenden Tabellen wiedergegeben.

### Beispiel 1

| Probe Nr. | Tier Nr. | Morgengemelk Zeit | Melatonin in pg/ml |
|---|---|---|---|
| 1 | 205 | 05:00 | 9,95 |
| 2 | 392 | 05:00 | 33,40 |
| 3 | 305 | 05:10 | 10,72 |
| 4 | 312 | 05:10 | 31,11 |
| 5 | 303 | 05:10 | 10,71 |
| 6 | 252 | 05:10 | 13,69 |
| 7 | 375 | 05:20 | 32,98 |
| 8 | 277 | 05:25 | 32,49 |
| 9 | 298 | 05:25 | 27,49 |
| 10 | 233 | 05:30 | 17,79 |
| 11 | 299 | 05:35 | 32,03 |
| 12 | 386 | 05:40 | 11,15 |
| 13 | 241 | 05:45 | 14,53 |
| 14 | 286 | 05:50 | 11,68 |
| 15 | 250 | 05:50 | 24,18 |
| 16 | 322 | 05:50 | 26,77 |
| 17 | 234 | 05:50 | 16,44 |
| 18 | 319 | 05:50 | 33,48 |
| 19 | 257 | 04:50 | 13,16 |
| 20 | 237 | 04:50 | 22,26 |
| 21 | 255 | 04:40 | 24,12 |
| 22 | 331 | 04:50 | 10,97 |
| 23 | 315 | 04:50 | 14,40 |
| 24 | 356 | 04:50 | 11,79 |
| 25 | 403 | 04:55 | 32, 08 |
| 26 | 280 | 04:55 | 10,62 |
| 27 | 254 | 04:55 | 11,35 |
| 28 | 212 | 05:50 | 9,73 |
| 29 | 276 | 05:30 | 32,14 |
| 30 | 344 | 06:10 | 11,80 |
| 31 | 309 | 06:10 | 34,11 |
| 32 | 316 | 05:10 | 22,14 |
| | | Durchschnitt | 20,35 |

### Beispiel 2

| Probe Nr. | Tier Nr. | Morgengemelk | Melatonin |
|---|---|---|---|
| | | Zeit | in pg/ml |
| 1 | 17 | 05:00 | 3,30 |
| 2 | 205 | 05:00 | 17,79 |
| 3 | 314 | 05:10 | 5,53 |
| 4 | 303 | 05:10 | 11,79 |
| 5 | 256 | 05:10 | 4,15 |
| 6 | 34 | 05:10 | 9,47 |
| 7 | 386 | 05:20 | 16,44 |
| 8 | 254 | 05:25 | 14,53 |
| 9 | 286 | 05:25 | 11,68 |
| 10 | 356 | 05:30 | 10,71 |
| 11 | 257 | 05:35 | 13,16 |
| 12 | 366 | 05:40 | 7,35 |
| 13 | 315 | 05:45 | 9,95 |
| 14 | 370 | 05:50 | 8,56 |
| 15 | 241 | 05:50 | 11,15 |
| 16 | 234 | 05:50 | 11,35 |
| 17 | 233 | 05:50 | 10,62 |
| 18 | 237 | 05:50 | 22,26 |
| 19 | 330 | 04:50 | 9,28 |
| 20 | 250 | 04:50 | 10,72 |
| 21 | 322 | 04:40 | 10,97 |
| 22 | 270 | 04:50 | 6,44 |
| 23 | 312 | 04:50 | 9,73 |
| 24 | 227 | 04:50 | 9,27 |
| 25 | 403 | 04:55 | 13,69 |
| 26 | 340 | 04:55 | 3,40 |
| 27 | 313 | 04:55 | 8,06 |
| 28 | 365 | 05:50 | 2,70 |
| 29 | 392 | 05:30 | 14,40 |
| 30 | 319 | 06:10 | 11,18 |
| 31 | 344 | 06:10 | 8,31 |
| 32 | 309 | 06:20 | 10,12 |
| | | Durchschnitt | 10,25 |

### Beispiel 3 (Vergleich)

| Probe Nr. | Tier Nr. | Morgengemelkzeit | Melatonin in pg/ml |
|---|---|---|---|
| 1 | 386 | 3.10 - 4.10 | 8,06 |
| 2 | 375 | 3.10 - 4.10 | 5,63 |
| 3 | 363 | 3.10 - 4.10 | 5,06 |
| 4 | 370 | 3.10-4.10 | 7,18 |
| 5 | 265 | 3.10 - 4.10 | 3,58 |
| 6 | 34 | 3.10-4.10 | 3,51 |
| 7 | 301 | 3.10-4.10 | 4,35 |
| 8 | 383 | 3.10 - 4.10 | 3,90 |
| 9 | 314 | 3.10 - 4.10 | 3,60 |
| 10 | 17 | 3.10-4.10 | 1,91 |
| 11 | 250 | 3.10 - 4.10 | 9,47 |
| 12 | 247 | 3.10 - 4.10 | 5.72 |
| 13 | 305 | 3.10 - 4.10 | 5,88 |
| 14 | 286 | 3.10 - 4.10 | 4,40 |
| 15 | 205 | 3.10-4.10 | 6,41 |
| 16 | 382 | 3.10-4.10 | 8,24 |
| 17 | 322 | 3.10-4.10 | 3,98 |
| 18 | 392 | 3.10-4.10 | 5,24 |
| 19 | 330 | 3.10 - 4.10 | 6,60 |
| 20 | 142 | 3.10 - 4.10 | 1,47 |
| 21 | 303 | 3.10-4.10 | 2,40 |
| 22 | 241 | 3.10 - 4.10 | 1,52 |
| 23 | 296 | 3.10-4.10 | 4,99 |
| 24 | 340 | 3.10-4.10 | 3,50 |
| 25 | 405 | 3.10-4.10 | 7,10 |
| 26 | 316 | 3.10-4.10 | 0,36 |
| 27 | 345 | 3.10-4.10 | 4,65 |
| 28 | 411 | 3.10 - 4.10 | 4,94 |
| 29 | 344 | 3.10-4.10 | 3,74 |
| 30 | 365 | 3.10 - 4.10 | 3,21 |
| 31 | 309 | 3.10-4.10 | 3,48 |
| 32 | 319 | 3.10-4.10 | 2,31 |
| | | Durchschnitt | 4,57 |

## Patentansprüche

1. Verfahren zur Herstellung von Milch mit erhöhtem Melatoningehalt oder Milcherzeugnissen davon, bei dem der Tagesrhythmus von einem oder mehreren weiblichen Säugetieren in eine Tagphase unter einem ersten Lichtregime mit Blaulichtanteil und in eine Nachtphase unter einem zweiten Lichtregime unterteilt wird und das oder die Tiere während der Nachtphase mindestens einmal gemolken werden, um Milch mit erhöhtem Melatoningehalt zu erhalten, **dadurch gekennzeichnet, dass** während der Nachtphase mindestens eine Lichtquelle für das Lichtregime eingesetzt wird, die Licht im Wellenlängenbereich von 500 nm oder mehr und im wesentlichen kein Licht im Wellenlängenbereich unter 500 nm emittiert.

2. Verfahren nach Anspruch 1, **dadurch gekennzeichnet, dass** die Lichtquelle während der Nachtphase im Wellenlängenbereich des sichtbaren Lichts mindestens ein Maximum über 550 nm aufweist.

3. Verfahren nach Anspruch 1 oder 2, **dadurch gekennzeichnet, dass** die für die Nachtphase verwendet Lichtquelle ein Lumineszenzstrahler ist.

4. Verfahren nach einem der Ansprüche 1 bis 3, **dadurch gekennzeichnet, dass** die für die Nachtphase verwendet Lichtquelle eine LED-Lampe oder eine Natriumdampflampe ist.

5. Verfahren nach Anspruch 4, **dadurch gekennzeichnet, dass** die LED-Lampe rotes, gelbes, orange- oder amberfarbenes Licht oder eine Mischfarbe davon emittiert, wobei eine rote LED-Lampe besonders bevorzugt ist.

6. Verfahren nach einem der Ansprüche 1 bis 5, **dadurch gekennzeichnet, dass** das Tier ein Schaf, eine Ziege oder eine Kuh ist.

7. Verfahren nach einem der Ansprüche 1 bis 5, **dadurch gekennzeichnet, dass** eine laktierende Tiergruppe unter diesem Tagesrhythmus gehalten wird.

8. Verfahren nach einem der Ansprüche 1 bis 7, **dadurch gekennzeichnet, dass** das Tier während eines 24-Stunden-Tags mindestens zweimal gemolken wird, wobei während der Tagphase gewonnene Milch nicht für die Milch mit erhöhtem Melatoningehalt verwendet wird.

9. Verfahren nach einem der Ansprüche 1 bis 8, **dadurch gekennzeichnet, dass** die Lichtquelle in der Nachtphase zumindest während des Melkvorgangs eingesetzt wird.

10. Verfahren nach einem der Ansprüche 1 bis 9, **dadurch gekennzeichnet, dass** die Lichtquelle während der Nachtphase mindestens 2 Stunden und vorzugsweise im wesentlichen während der ganzen Nachtphase eingesetzt wird.

11. Verfahren nach einem der Ansprüche 1 bis 10, **dadurch gekennzeichnet, dass** für das Lichtregime der Tagphase eine oder mehrere Vollspektrumlampen, Sonnenlicht oder andere Lichtquellen mit hoher circadianer Wirkung eingesetzt werden.

12. Verfahren nach einem der Ansprüche 1 bis 11, **dadurch gekennzeichnet, dass** die Tagphase länger als 14 h dauert.

13. Verfahren nach einem der Ansprüche 1 bis 12, **dadurch gekennzeichnet, dass** der Milch Lactose entzogen wird und/oder eine Fettreduktion der Milch durchgeführt wird.

14. Verfahren nach einem der Ansprüche 1 bis 13, **dadurch gekennzeichnet, dass** die in der Nachtphase verwendete Lichtquelle eine Beleuchtungsstärke von mehr als 50 lux, bevorzugt mehr als 100 lux ergibt.

15. Verfahren nach einem der Ansprüche 1 bis 14, **dadurch gekennzeichnet, dass** die mit Melatonin angereicherte Milch zu Milchpulver verarbeitet wird.

16. Verfahren nach einem der Ansprüche 1 bis 15, **dadurch gekennzeichnet, dass** die Milchleistung der laktierenden Tiere erhöht wird.

17. Milchpulver mit einer Melatoninkonzentration von mehr als 150 pg/g, bei dem das natürliche Melantonin an Milcheiweiß gebunden ist, erhältlich nach dem Verfahren von Anspruch 15.

18. Verwendung des Milchpulvers nach Anspruch 17 als oder für ein Nahrungsmittel oder Nahrungsergänzungsmittel.

## Claims

1. A method for the production of milk with increased melatonin content or milk products of it, in which the daily cycle of one or more female mammals is divided into a daytime phase under a first light regime with a proportion of blue light and a night-time phase under a second light regime and the animal or animals are milked at least once during the night-time phase in order to obtain milk with an increased melatonin content, **characterised in that** during the night-time phase at least one light source is used for the light regime, which emits light in the wavelength range of 500 nm or more and essentially no light is emitted in the wavelength range below 500 nm.

2. The method according to claim 1, **characterised in that** the light source exhibits at least a maximum above 550 nm in the wavelength range of visible light during the night-time phase.

3. The method according to claim 1 or 2, **characterised in that** the light source used for the night-time phase is a luminescent emitter.

4. The method according to one of claims 1 to 3, **characterised in that** the light source used for the night-time phase is an LED lamp or a sodium vapour lamp.

5. The method according to claim 4, **characterised in that** the LED lamp emits red, yellow, orange or amber-coloured light or a mixed colour of these, wherein a red LED lamp is particularly preferred.

6. The method according to one of claims 1 to 5, **characterised in that** the animal is a sheep, goat or cow.

7. The method according to one of claims 1 to 5, **characterised in that** a lactating animal group is kept under this daily cycle.

8. The method according to one of claims 1 to 7, **characterised in that** the animal is milked during a 24-hour day at least twice, wherein milk obtained during the daytime phase is not used for the milk with increased melatonin content.

9. The method according to one of claims 1 to 8, **characterised in that** the light source is used in the night-time phase at least during the milking process.

10. The method according to one of claims 1 to 9, **characterised in that** the light source during the night-time phase is used for at least two hours and preferably essentially during the complete night-time phase.

11. The method according to one of claims 1 to 10, **characterised in that** for the light regime in the daytime phase one or more full-spectrum lamps, sunlight or other light sources with a high circadian effect are used.

12. The method according to one of claims 1 to 11, **characterised in that** the daytime phase lasts longer than 14 h.

13. The method according to one of claims 1 to 12, **characterised in that** lactose is extracted from the milk and/or fat reduction of the milk is carried out.

14. The method according to one of claims 1 to 13, **characterised in that** the light source used in the night-time phase produces an illuminance of more than 50 lux, preferably more than 100 lux.

15. The method according to one of claims 1 to 14, **characterised in that** the milk enriched with melatonin is processed to form milk powder.

16. The method according to one of claims 1 to 15, **characterised in that** the milk capacity of the lactating animals is increased.

17. A milk powder with a melatonin concentration of more than 150 pg/g, wherein the natural melatonin is bound to milk protein, obtainable according to the method of Claim 15.

18. Use of the milk powder according to claim 17 as or for a foodstuff or nutritional supplement.

## Revendications

1. Procédé pour la production de lait ou de produits laitiers à haute teneur en mélatonine, dans lequel le rythme journalier d'un ou de plusieurs mammifère(s) femelle(s) est décomposé en une phase diurne avec un premier régime de lumière bleue et en une phase nocturne avec un deuxième régime de lumière, ou de sorte que les animaux soient traits au minimum une fois pendant la phase nocturne, afin d'obtenir du lait avec une haute teneur en mélatonine, procédé **caractérisé en ce qu'**au moins une source lumineuse est employée pour le régime de lumière pendant la phase nocturne, émettant une lumière dans le domaine de longueur d'onde de 500 nm ou plus et n'émettant essentiellement pas de lumière dans le domaine de longueur d'onde en dessous de 500 nm.

2. Procédé selon la revendication 1 **caractérisé en ce que** la source lumineuse présente au moins un maximum au-dessus de 550 nm dans le domaine de longueur d'ondes de la lumière visible pendant la phase nocturne.

3. Procédé selon la revendication 1 ou 2, **caractérisé en ce que** la source lumineuse qui est utilisée pour la phase nocturne est un spot de luminescence.

4. Procédé selon l'une des revendications 1 à 3, **caractérisé en ce que** la source lumineuse qui est utilisée pour la phase nocturne est une lampe LED ou une lampe à vapeurs de sodium.

5. Procédé selon la revendication 4 **caractérisé en ce que** la lampe LED émette une lumière rouge, jaune, orange ou ambre ou un mélange de ces couleurs, une lampe LED rouge étant particulièrement préférée.

6. Procédé selon l'une des revendications 1 à 5, **caractérisé en ce que** l'animal est une brebis, une chèvre ou une vache.

7. Procédé selon l'une des revendications 1 à 5, **caractérisé en ce qu'**un groupe d'animaux en lactation est maintenu sous ce rythme diurne.

8. Procédé selon l'une des revendications 1 à 7, **caractérisé en ce que** l'animal est trait au moins deux fois pendant une journée de 24 heures, le lait récolté durant la phase diurne n'étant pas employé pour le lait à haute teneur en mélatonine.

9. Procédé selon l'une des revendications 1 à 8, **caractérisé en ce que** la source lumineuse employée pendant la phase nocturne est employée au minimum pendant la traite.

10. Procédé selon l'une des revendications 1 à 9, **caractérisé en ce que** la source lumineuse de la phase nocturne est employée pendant au moins 2 heures et de préférence essentiellement pendant la phase nocturne entière.

11. Procédé selon l'une des revendications 1 à 10, **caractérisé en ce qu'**une ou plusieurs lampe(s) à spectre complet, la lumière solaire ou d'autres sources lumineuses à forte influence circadienne sont employées pour le régime de lumière de la phase diurne.

12. Procédé selon l'une des revendications 1 à 11, **caractérisé en ce que** la phase diurne dure plus de 14 heures.

13. Procédé selon l'une des revendications 1 à 12, **caractérisé en ce que** du lactose est prélevé sur le lait et/ou qu'une réduction de matière grasse est effectuée sur le lait.

14. Procédé selon l'une des revendications 1 à 13, **caractérisé en ce que** la source lumineuse employée pendant la phase nocturne délivre un éclairement de plus de 50 lux, de préférence de plus de 100 lux.

15. Procédé selon l'une des revendications 1 à 14, **caractérisé en ce que** le lait enrichi en mélatonine est transformé en lait en poudre.

16. Procédé selon l'une des revendications 1 à 15, **caractérisé en ce que** le rendement en lait des animaux en lactation est augmenté.

17. Lait en poudre avec une concentration en mélatonine supérieure à 150 pg/g, dans lequel de la mélatonine naturelle est liée à la protéine de lait, obtenu selon le procédé de la revendication 15.

18. Emploi du lait en poudre selon la revendication 17 soit en tant qu'aliment, soit en tant qu'aliment complémentaire.
